# EUROPEAN PATENT SPECIFICATION

(11) **EP 0 610 778 B1**
(45) Date of publication and mention of the grant of the patent: **24.05.2000**
(21) Application number: 94101464.9
(22) Date of filing: 01.02.1994
(51) Int. Cl.: A61M 1/00, A61M 1/02

(54) **Liquid separating apparatus**
Vorrichtung zum Trennen von Flüssigkeiten
Appareil de séparation de liquides

(30) Priority: 03.02.1993 JP 1664793
(43) Date of publication of application: 17.08.1994
(73) Proprietor: TERUMO KABUSHIKI KAISHA, Tokyo (JP)
(72) Inventor: Misumi, Masashi, c/o Terumo Kabushiki Kaisha, Fuji-shi, Shizuoka-ken (JP)
(74) Representative: Casalonga, Axel

(56) References cited:
- EP-A- 0 416 495
- EP-A- 0 484 751
- US-A- 4 350 585

## Description

This invention relates to a liquid separating apparatus for individually extracting a plurality of different liquids from such a flexible container as the blood bag which is separated into a layer of different components.

Recently, the componential transfusion which consists in transfusing only necessary components of blood to a patient has been prevailing in the place of the whole blood transfusion and the exclusive extraction of plasma from the blood for the preparation of plasma medicines has been in popular. For the purpose of obtaining the blood for use in the componential transfusion or the preparation of plasma medicines, the flexible blood bag or the so-called multiple-bag has been used so far. The blood bag container comprises a blood collecting bag (so-called primary bag) for storing the blood collected from the donor and one or more separation bags (so-called satellite bags) communicating via liquid extracting tubes (hereinafter referred to simply as "tubes") with the primary bag. The centrifugally separated components of blood in the primary bag are individually extracted and admitted into the satellite bags.

When the blood collecting bag is treated with a centrifuge, the blood in the bag is separated into such layers of different components as a plasma layer, a red blood cell layer and an intermediate layer (a soft membranous layer containing platelets, white blood cells, etc., so-called buffy coat layer). For these components of blood to be utilized afterward as separated, it is necessary that these components should be collected independently of each other from the blood collecting bag and stored respectively in the separation bags so as to preclude the separated components from being commingled.

The fractional collection of such components of blood has been heretofore accomplished by abuttting the blood bag to a liquid separating device and then compressing the blood bag with pressure externally exerted on the opposite surfaces thereof thereby allowing the separated components to be transferred into the relevant separation bags through the relevant component extracting tubes which communicate with the blood bag.

By means of the blood bag of the kind which, as disclosed in JP-A-3-47,266 (EP 407,999 A), is provided in the upper part thereof with blood collecting tubes or component extracting tubes, however, the componential extraction of blood is not easily attained. Particularly when the intermediate layer is to be extracted into the satellite bag, the blood bag has a problem of controllability in choosing suitable timing for extracting the intermediate layer. Further, since the intermediate layer is liable to adhere to the inner wall of the primary bag, it is difficult to extract this intermediate layer thoroughly from the primary bag.

In the case of the blood bag which, as disclosed in JP-B-63-20,144 (US 4,608,178) , is provided in the upper and lower parts of the primary bag with tubes, the componential extraction is attained rather easily. Since the ports to be used for the extraction are disposed at the upper and lower edges of the bag proper, however, the tubes are not handled conveniently during the attachment of the bag to the centrifuge or the blood component separating device.

In the case of the blood bag which, as disclosed in EP-A-0484751A1 (US 5,154,716) , is provided with a separation path, ie. a conduit formed in part of the interior of the primary bag by bonding or fusion of opposed narrow portions of the inner wall surfaces of the bag, for guiding the liquid of the lower layer to the upper part of the bag proper, the accuracy of the collection of the red blood cells is decreased in proportion as the width of the path is increased because parts of the plasma component and the intermediate layer component remain in the path besides part of the red blood cells of the lower layer subsequently to the step of centrifugal separation. If the width of the path is decreased for the purpose of exalting the accuracy of collection, the uncomfortable possibility arises that the required time for the extraction increases and the speed of flow of the blood through the path immensely increases and the possibility for destorying the red blood cell. If the path is given a more or less sizable width, the accuracy of collection of the red blood cell component is lowered. When the components of blood which have been fractionally collected by the blood bag provided with this path of the sizable width are used, there ensues the necessity for depriving these components of impurities with the aid of such an instrument as a white blood cell removing filter or a varying adsorbent, for example. The measure of thermally fusing the opposed walls of the primary bag necessitates formation of a sealed part which extends to the bottom part of the bag and results in an increase in the number of process steps. During the attachment of the primary bag to the blood separation device, the convenience with which the primary bag is handled is impaired because the obverse and reverse sides of the primary bag are fixed when this bag is attached to the blood separation device.

By the manner of compressing the primary bag which has undergone centrifugal separation, the liquid separating devices are classified into those of the lower biased compression type disclosed in JP-B-55-17,585 and those of the parallel compression type disclosed in JP-A-55-155,652 (US 4,350,585) . The devices of the former type are intended to operate by suspending a primary bag from a hook provided on a separation stand and compressing the suspended primary bag with pressure externally exerted on the opposite walls of the bag by means of a pressing plate which is so hinged as to render the lower end thereof pivotable and the devices of the latter type are operated by placing a pressing plate parallelly to a separation stand and interposing a primary bag between the separation stand and the pressing plate and pressing the pressing plate across the interposed bag against the separation stand with pressure externally exerted on the opposite walls of the bag.

Some of these liquid separating devices are adapted to effect the pressurization automatically or manually. The liquid separating devices capable of automatic pressurization which have been recently finding extensive utility, without discrimination between the two types mentioned above, are intended to operate by continuing detection of the position of an intermediate layer by means of an interface sensor and meanwhile compressing the bag by means of a pressing plate.

In the case of the devices of the lower biased compression type, since detecting the position of the intermediate layer inside the primary bag during the pressurization to the bag is achieved by detecting the part of the bag having a large cross section, the possibility arises that even a slight error in the position of detection notably affects the accuracy of fractionation of the blood components and impairs the accuracy of fractionation. The devices of the parallel compression type are at a disadvantage in rendering difficult and complicate the control which is to be exercised when the pressing plate is actuated translationally so as to exert uniform pressure to the bag.

As an improvement to eliminate these drawbacks of the conventional liquid separating devices, a device of the upper compression type disclosed in JP-A-2-98,362 (EP-348,682 A) has been proposed. The device of this upper biased compression type requires the pressing plate to have its fulcrum in the upper part thereof and, therefore, is at an advantage in having a small cross sectino in the part thereof used for upward extraction and also involving only a small error in the detection of the position of the intermediate layer in the blood bag. Since this device is obliged to continue detection of the constantly changing position of the intermediate layer, it inevitably complicates the configuration for the overall control of the device. Moreover, since the pressing plate uses such a drive unit as a motor which has a relatively heavy weight, the device as a whole has a great weight and costs much.

US-A-4,608,178 upon which the preamble of claim 1 is based relates to a method and a blood bag system for the separation of blood components in which the blood is tapped into a first blood bag having outlets both at the top and at the bottom and in which after centrifugation the upper plasma layer is passed out through the upper outlet and the red cell lower layer is passed out through the lower outlet, the level of the intermediate buffy coat layer being detected by sensing means to open/shut off the outlets in connection with the detected level of the intermediate layer.

The present invention, produced for the elimination of the drawbacks suffered by the conventional devices as described above, has for its object the provision of a liquid separating apparatus which operates by alternately extracting a plasma layer and a red blood cell layer at a predetermined interval thereby minimizing the motion of the intermediate layer in the vertical direction and allowing the components of blood to be extracted in one direction with high controllability.

A further object of this invention is to provide a liquid separating apparatus which effects separation of the blood components with high accuracy.

Another object of this invention is to provide a liquid separating apparatus using tubes which are easy to handle.

Yet another object of this invention is to provide a liquid separating apparatus which is compact and inexpensive.

To accomplish these objects, this invention is directed to a liquid separating apparatus comprising a container retaining member for retaining a flexible parent container adapted to store therein components fractionated into at least three layers, ie. an upper layer, an intermediate layer and a lower layer, said flexible parent container being provided on a side thereof facing either of the upper layer or the lower layer with a first outlet and a second outlet; a container pressing member comprising a supporting member for supporting either one of two opposed surfaces of the parent container and a pressing member for pressing the other surface of the parent container ; an interface detecting member for detecting the position of the liquid level of the intermediate layer in the container during the pressurization of the parent container by the container pressing member ; characterized in that it further comprises:
- a first clamp member and a second clamp member for independently blocking the flows of liquids generated from said parent container and through said first and second outlets due to pressurization of the parent container by said pressing member ; and
- a control unit for controlling the open-shut states of said first and second members according to signals from said interface detecting member, thereby keeping the position of said intermediate layer at a substantially fixed level and guiding said upper layer and said lower layer to the predetermined outlets, and a weight measuring unit for measuring the weight of said parent container; said control unit, before pressurization, comparing the weight of said parent container measured by weight measuring unit to a first predetermined weight for allowing pressurization when the weight of said parent container equals the first predetermined weight, and during the depression of said container by said container pressing member comparing the weight of said parent container continuously measured by weight measuring unit to a second predetermined weight to effect the open-shut control of said clamp members based on the data of the weight of said parent container and the position of said intermediate layer, and shutting the two clamp members to stop the flows of liquids when a predetermined time has elapsed or the weight of said parent container equals the second predetermined weight.

The other objects, features, and advantages of this invention will become apparent when consideration is given to the following detailed description thereof, which makes reference to the annexed drawings wherein :
Fig. 1 is a perspective view schematically illustrating a liquid separating apparatus as one embodiment of this invention.
Fig. 2 is a plan view illustrating one example of a blood bag.
Fig. 3 is a cross section taken through Fig. 1 along the line 3-3.
Fig. 4 is a cross section taken in a horizontal plane to illustrate the essential part of the apparatus of Fig. 1.
Fig. 5 is an explanatory diagram illustrating the operation of pressurization by a container pressing unit.
Fig. 6 is an explanatory diagram illustrating the container pressing unit in the unlocked state.
Fig. 7 is a flow chart of the embodiment.
Fig. 8 is a front view illustrating a primary bag of another type suspended from hooks.

Now, one embodiment of this invention will be described below with reference to the accompanying drawings.

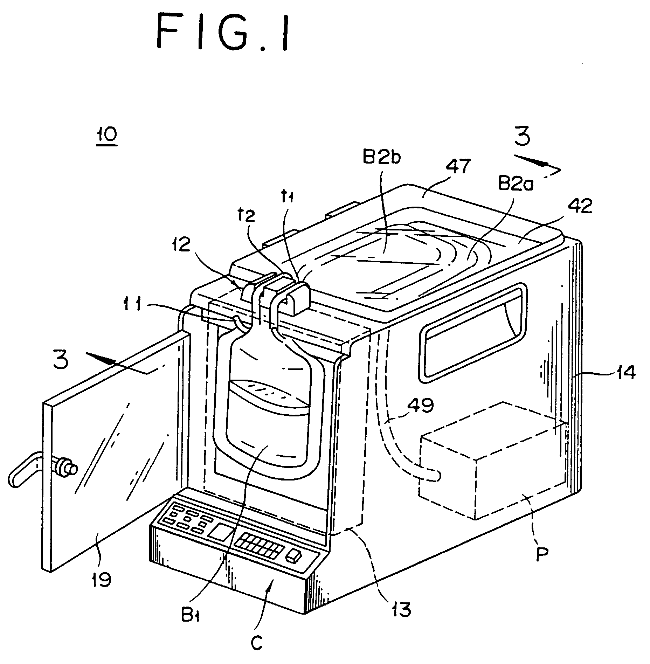

In Fig. 1, a liquid separating apparatus 10 fulfills the role of individually extracting components of blood from a parent container or a blood collecting bag (hereinafter referred to simply as "primary bag") B1 which has undergone the treatment of centrifugal separation and this apparatus 10 comprises a container retaining member such as a hook 11 for retaining the primary bag B1, a clamp unit 12 for selectively clamping tubes t1, t2, a container pressing unit 13 for pressing the primary bag B1 supported by the hooks 11, and a control unit C for controlling the container pressing unit 13, etc.

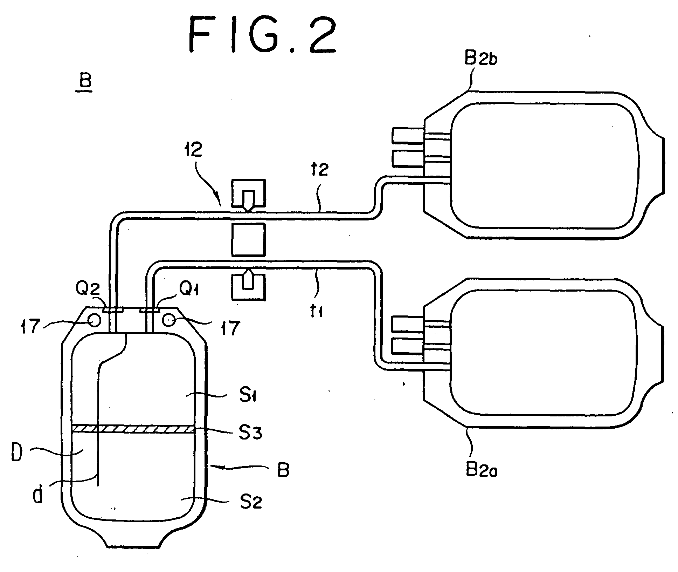

The blood bag assembly B is made of a flexible synthetic resin and is provided, as illustrated in Fig. 2, with a blood collecting bag or the so-called primary bag B1 for storing the blood collected from a donor. In the upper part of the primary bag, these are provided a first outlet port Q1 and a second outlet port Q2 (corresponding to the first and the second outlet). To the outlet ports Q1, Q2 are respectively connected the tubes t1, t2. To the primary bag B1, one of a plurality of separation bags, a so-called satellite bag B2a is connected via the tube t1 and another satellite bag B2b is connected via the second outlet port Q2. The primary bag B1 is provided with a partition wall d and this partition wall d defines an inner path D. When the blood in the primary bag B1 is subjected to centrifugal separation, it is divided into a plasma layer S1 which is formed in the upper part, a red blood cell layer S2 which is formed in the lower part and an intermediate layer (buffy coat layer) S3. It is the plasma layer S1 and the red blood cell layer S2 that are used. The useful components of blood are independently extracted from the primary bag B1, specifically the component of the plasma layer S1 is extracted via the tube t1 and introduced into the satellite bag B2a and the component of the red blood cell layer S2 is extracted via the tube t2 and introduced into the satellite bag B2b preparatorily containing a preserving liquid for red blood cell.

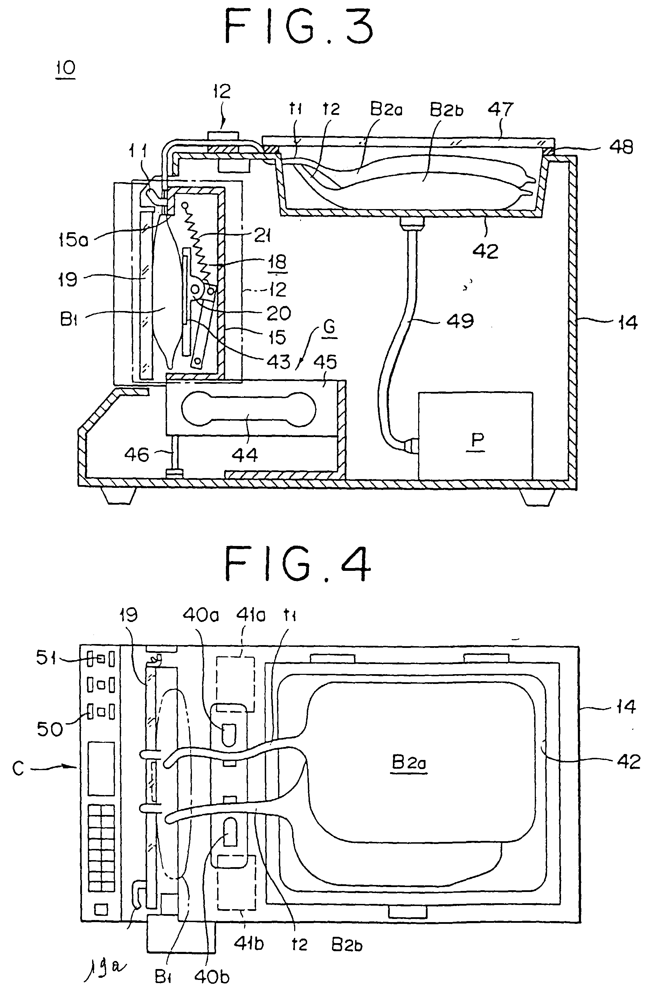

The container pressing unit 13 mentioned above is disposed, as illustrated in Fig. 1 and Fig. 3, inside a housing 15 which is inserted in the front part of the main body case 14. A pair of hooks 11 which constitute the container retaining member project from a front wall 15a of the housing 15. The primary bag B1 is suspended by means of holes 17 formed in the primary bag B1 inserted around the hooks 11.

The housing 15 of the container pressing unit 13 is attached to an aluminum block 45 which will be described specifically afterward and is provided, as illustrated in Fig. 3, with a container pressing member 18 which is adapted to apply pressure from the front surface side and the rear surface side to the primary bag B1 suspended from the hooks 11.

This container pressing member 18 is provided with a first pressing plate 19 (supporting member) adapted to support the primary bag B1 by approaching and contacting with the front surface side thereof and, at the same time, maintain a stationary state while pressing on the primary bag B1, a second pressing plate 20 (pressing member) adapted to approach and contact with the rear surface side of the primary bag B1 and press thereon, and a spring member 21 adapted to generate pressure in a direction causing the second pressing plate 20 to press the primary bag B1 or urging the second pressing plate 20 toward the first pressing plate 19.

In the present embodiment, since the first pressing plate 19 is formed of a door member which opens and shuts the front surface part of the housing 15, it avoids giving an undesirable addition to the number of component parts of the apparatus and contributes to simplification of the construction of the apparatus. Thus, the apparatus is compact and inexpensive.

This door member is preferably made of a transaprent material such as acrylic resin so as to permit visual observation of the primary bag B1 which is disposed behind the door member.

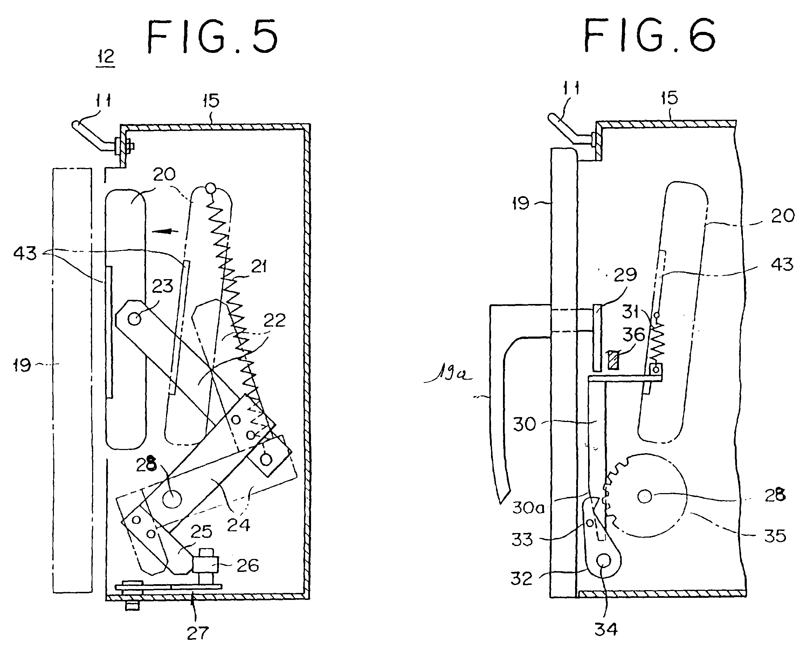

The second pressing plate 20 is disposed inside the housing 15 as opposed to the first pressing plate 19. To the rear surface side of the second pressing plate 20, a forward end of a first link 22 is pivotably connected by means of a pin 23 as illustrated in Fig.5. To the rear end of the first link 22 is connected an end part of a second link 24 which is pivotably supported on a supporting shaft 28 attached to the housing 15. An end part of a third link 25 is attached to the other end part of the second link 24 and the other end part of the third link 25 is kept in contact with a pressing member 26. The pressing member 26 is connected to the door member through a link mechanism 27 and forces the second pressing plate 20 backward agains the biasing force of the spring member 21 as a result of the release of the door member. It is for the purpose of decreasing the number of component pans of apparatus and simplifying the construction of apparatus and further utilizing the opening-shutting force of the door member for forcing the second pressing plate 20 backward and enabling the container pressing member 18 to assume a state ready for pressurization as a result of the release of the door member that the door member is formed as one member of the container pressing member 18 and serves concurrently as the first pressing member 19, thus obviating the necessity of providing an independent pressing plate. The preparation of pressurization of the primary bag B1 is completed without delay after releasing the door member.

The first pressing plate 19 which is in the form of a door member is provided, as illustrated in Fig. 6, with a handle 19a. This handle 19a is provided at the end part thereof with a cam member 29. Thus, a rotation of the handle 19a releaves the second pressing plate 20 of its locked state. In other words, when the handle 19a is rotated, the cam member 29 is actuated to depress a push bar 30 against the biasing force of an urging member 31 such as a spring member. Since the urging member 21 is actuated to generate its biasing force when the handle 19a is locked as described above, the primary bag B1 can be immediately pressed by setting the primary bag B1.

The push bar 30 has a tapered member 30a formed at the leading end part thereof This tapered member 30a is adapted to engage a pin 33 attached to a claw member 32. The claw member 32 is urged in one direction about a pin 34 as by means of an urging member such as spring member (not shown) and interlocked with a ratchet 35 attached to the supporting shaft 28 and consequently caused to lock this ratchet 35. The second link 24 attached to the supporting shaft 28 is unlocked and enabled to rotate freely by causing the push bar 30 mentioned above to push pin 33 and retracting the claw member 32 interlocked with the ratchet 35. As a result, the urging member 21 takes its own course of causing the second pressing plate 20 to press on the primary bag B1. In Fig. 6, the reference numeral 36 stands for a stopper.

In the upper part of the main body case 14 mentioned above, the clamp unit 12 mentioned above independently effects a block-unblock control of the flows of blood components inside the two tubes t1, t2 extended from one end part of the primary bag B1 as illustrated in Fig. 1 and Fig. 4. This clamp unit 12 is provided with a first clamp member 40a for blocking-unblocking the tube t1 passing the plasma component therein, a second clamp member 40b for blocking-unblocking the tube t2 passing the red blood cell component therein, and solenoids 41a, 41b for actuating the clamp members 40a, 40b and is adapted to effect the block-unblock control in response to the signals output from the control unit C in accordance with the position of the intermediate layer S3 of the centrifugally separated blood, the weights of the primary bag B1 and/or satellite bags B2a, B2b, and the duration of pressurization. The components of blood which have flowed through the tubes t1, t2 are stored in the satellite bags B2a, B2b which are disposed inside a tray 42 formed in the upper part of the main body case 14.

The position of the intermediate layer S3 is detected by an interface detecting member 43 which is disposed in the second pressing plate 20. A photosensor is substantially used as the interface detecting member 43 and is adapted to detect the interface on the basis of the differences of the aforementioned three layers in terms of light absorbance. The signals which are issued from this interface detecting member 43 are input into the control unit C.

A weight measuring unit G, as illustrated in the diagram, is formed of a weight sensor 44 which is disposed in the lower part of the container pressing unit 13 and is adapted to calculate the weight of the primary bag B1 by subtracting the tare such as of the housing 15 and the first and second pressing plates 19, 20 from the total weight of the container pressing unit 13 mentioned above. In other words, the total weight of the container pressing unit 13 is detected by the weight sensor 44 which is provided in the lower part of the container pressing unit 13. When the weight of the primary bag B1 determined by the weight measuring unit G equals the second predetermined weight, the operation of the second pressing plate 20 is stopped and, at the same time, the tubes t1, t2 are clamped by the clamp members 40a, 40b to stop the flow of the blood components through the first outlet port Q1 and the second outlet port Q2. This weight sensor 44 may be formed of a strain gauge which is attached to the lateral side of the block 45 which is provided so as to support the housing 15 of the container pressing unit 13. Since the container pressing unit is not provided with a weighty drive unit such as a motor and, therefore, has a relatively small total weight, the determination of the weight can be attained with high accuracy by the use of an instrument having a small range of determination. In the diagram, the referential numeral 46 stands for a balance stopper.

The tray 42 mentioned above is closed with an upper lid 47, tightly sealed along the periphery thereof with a packing 48, and evacuated by the use of a pump P and a conduit 49. The tray 42 is intended to facilitate the inflow of the blood components from the primary bag B1 to the satellite bags B2a, B2b.

The duration of pressurization mentioned above is detected by a timer 51 provided on a control panel 50 which is installed on the front surface of the main body case 14. The signals from this timer 51 are likewise input into the control unit.

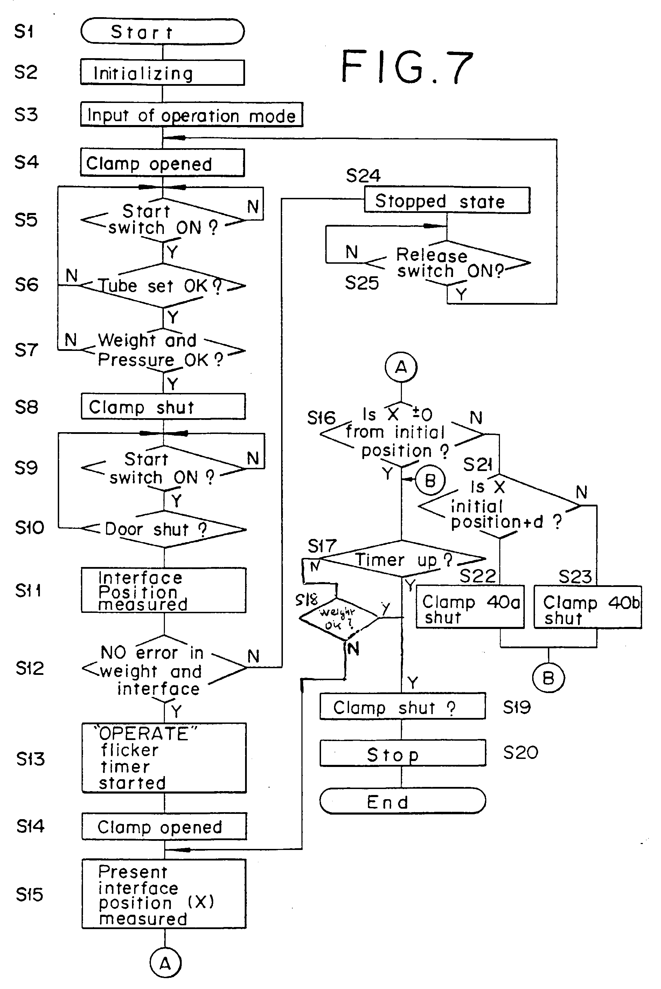

Now, the operation of the present embodiment will be described below with reference to a flow chart of Fig. 7.

First the primary bag B1 is set in place inside the housing 15 of the container pressing unit 13. Then, the power supply for the control unit C is turned on to start the program (S1). As a result, the memory is initialized and the weight measuring unit and the pressure measuring unit are reset (S2). When the predetermined operation mode and other data are input (S3), the clamp unit assumes an "opened" state (S4).

At this point, the primary bag B1 is in a state of the centrifugal separation generated by a centrifuge and having the blood already separated into three layers therein. The setting of the bag is carried out by a procedure which comprises inserting the through holes 17 of the primary bag B1 around the hooks 11, inserting the tubes t1, t2 extended from the primary bag B1 respectively into the clamp members 40a, 40b and, at the same time, placing the satellite bags B2a, B2b in the tray 42, and shutting the upper lid 47.

Then, the start switch is turned on as shown at Step 5. The two clamp members 40a, 40b are to be shut by the solenoids 41a, 41b (S8) when the start switch is turned on, the tubes t1, t2 are set in place normally, the weight of the primary bag B1 equals the first predetermined weight, the vacuum pump P is actuated, and the interior of the tray 42 is in a fully evacuated state. When the tubes t1, t2 are not normally set or when the weight of the primary bag B1 does not equal the first predetermined weight, the two clamp members 40a, 40b are not actuated because the condition of the apparatus is operationally incompetent.

When the primary bag B1 and other relevant parts are in their normal states, the start switch is turned on again as shown at Step 9. The weight of the primary bag B1 is calculated by subtracting the tare such as of the housing 15 and the first and second pressing plates 19, 20 from the total weight of the container pressing unit 13. In other words, the calculation of this weight is attained by subtracting the tare weight of the container pressing unit 13 input in advance in the control unit C memory from the weight of the container pressing unit 13 detected by the weight sensor 44. The calculated weight thus equals the first predetermined weight, the first pressing plate 19 in the form of a door member is shut (S10). When the weight of the primary bag B1 does not equal the first predetermined weight, the primary bag B1 is ejected and removed because the condition, of the apparatus is operationally incompetent.

In the primary bag B1 which has undergone the step of weight check as described above, the initial position of the interface is detected by the interface sensor. When both of these factors are normal state , the timer is started and the operation start is displayed (S13).

In this case, the pressurization is started by shutting the first pressing plate 19 and then rotating the handle 19a. The rotation of the handle 19a causes the cam member 29 to depress the pushing bar 30 against the biasing force of the urging member 31 such as coil spring member, brings the tapered member 30a at the leading end of the pushing bar 30 into engagement with the pin 33 of the claw member 32, breaks engagement between the claw member 32 and the ratchet 35, unlocks the second link 24 attached to the supporting shaft 23 and allows it to rotate freely, and causes the urging member 21 to press the second pressing plate 20 against the primary bag B1.

In this case, the second pressing plate 20 is so set as to exert a compressive force in the direction pressing the primary bag B1, the compressive force is intense in the initial state of pressurization and is gradually lowered with the advance of the flow of liquid and consequently the flow of the intermediate layer decreases proportionately.

Incidentally, the primary bag B1 is squeezed from the front surface side and the rear surface side thereof between the first pressing plate 19 and the second pressing plate 20. This state of compression of the primary bag B1 can be visually observed by the operator because the door member is made of a transparent material.

When the pressurization on the primary bag B1 is started, the position of the intermediate layer S3 is constantly detected by the interface detecting member 43 (S15) and compared with the initial position thereof (S16).

The open-shut states of the two clamp members 40a, 40b are allowed to continue so long as the result of determination by the interface detecting member 43 remains within the predetermined range of the initial value X0 ±a (wherein +a represents an upward displacement of the position of the intermediate layer S3 and -a a downward displacement of the position of the intermediate layer S3).

If the position of the intermediate layer S3 in the primary bag B1 is over +a, for example, the clamp member 40a for the tube t1 passing the plasma layer S1 is shut (S22) because the amount of the flow of the upper plasma layer S1 is unduly large. Conversely, if the position of the intermediate layer S3 in the primary bag B1 is under -a, the clamp member 40b of the tube t2 passing the red blood cell layer S2 is shut (S23) because the amount of the flow of the lower red blood cell layer S2 is unduly large.

The apparatus, thus, allows the flows of blood components to proceed and meanwhile effects due control of the flows so that the intermediate layer S3 constantly remains substantially at the predetermined position inside the primary bag B1.

In the manner described above, the two clamp members 40a, 40b are suitably opened and shut and, as a result, the plasma layer S1 or the red blood cell layer S2 in the primary bag B1 is forwarded to the satellite bag B2a through the tube t1 or to the satellite bag B2b containing the preserving agent for the red blood cell through the tube t2 respectively.

When a predetermined time elapses thence (S17) or when the weight of the primary bag B1 reaches the second predetermined value (S18), the two clamp members 40a, 40b are shut (S19) and the flows of the blood components are stopped (S20).

After the blood components have been extracted alternately from the primary bag B1 and delivered to the satellite bags B2a, B2b as described above, the tubes t1, t2 are shut as with clips, the door member is opened, and the primary bag B1 and the satellite bags B2a, B2b are removed from the liquid separating apparatus.

In the present embodiment, when the door member is opened at this point, the second pressing plate 20 retracts and the container pressing member 18 assumes a state ready for pressurization. In other words, the primary bag B1 can be immediately readied for pressurization by opening the door member. In consequence of the opening of the door member, the third link 25 is depressed through the link mechanism 27, the second pressing plate 20 is retracted against the biasing force of the spring member 21, and the ratchet 35 is interlocked with the claw member 32 to be locked. When the separation of the liquids is completed and the door member is opened as described above, the second pressing plate 20 of the container pressing member 18 is caused to move in the direction opposite to the direction of pressurization. This arrangement not only facilitates the setting of the primary bag B1 but also exalts the operability of the apparatus.

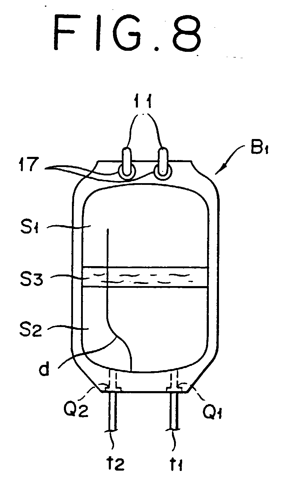

The present embodiment is adapted for the primary bag B1 which is provided in the upper part thereof with the two outlet ports Q1, Q2. Alternatively, the present invention can be easily adapted for a primary bag B1 which is intended to be suspended from hooks 11 as illustrated in Fig. 8, namely a primary bag B1 which is provided in the lower part thereof with both outlet ports Q1, Q2. When the outlet ports are provided on the lower side of the primary bag B1 as described above, this primary bag B1 is allowed to utilize gravitational attraction for the discharge of the blood components.

While the embodiment cited above represents a case of using a blood bag and collecting components of blood fractionally, this invention can be used for fractional collection of various liquids besides components of blood.

## Claims

1. A liquid separating apparatus comprising :
- a container retaining member (11) for retaining a flexible parent container (B1) adapted to store therein components fractionated into at least three layers, i.e. an upper layer (S1), an intermediate layer (S3) and a lower layer (S2), said flexible parent container (B1) being provided on a side thereof facing eithe of said upper layer (S1) or of said lower layer (S2) with a first outlet (Q1) and a second outlet (Q2),
- a container pressing member (18) comprising a supporting member (19) for supporting either one of two opposed surfaces of said parent container (B1) and a pressing member (20) for pressing the other surface of said parent container (B1) ;
- an interface detecting member (43) for detecting a position of the liquid level of said intermediate layer (S3) in said parent container (B1) during pressurization of said parent container by said container pressing member (18), characterized in that it further comprises:
- a first clamp member (40a) and a second clamp member (40b) for independently blocking the flows of liquids generated from said parent container and through said first and second outlets (Q1, Q2) due to pressurization of the parent container (B1) by said pressing member (20) ; and
- a control unit (C) for controlling the open-shut states of said first and second members (40a, 40b) according to signals from said interface detecting member (43), thereby keeping the position of said intermediate layer (S3) at a substantially fixed level and guiding said upper layer (S1) and said lower layer (S2) to the predetermined outlets (Q1, Q2), and a weight measuring unit (G) for measuring the weight of said parent container (B1); said control unit (C), before pressurization, comparing the weight of said parent container (B1) measured by weight measuring unit (G) to a first predetermined weight for allowing pressurization when the weight of said parent container (B1) equals the first predetermined weight, and during the depression of said container by said container pressing member (18) comparing the weight of said parent container (B1) continuously measured by weight measuring unit (G) to a second predetermined weight to effect the open-shut control of said clamp members (40a,40b) based on the data of the weight of said parent container (B1) and the position of said intermediate layer (S3), and shutting the two clamp members (40a, 40b) to stop the flows of liquids when a predetermined time has elapsed or the weight of said parent container (B1) equals the second predetermined weight.

2. A liquid separating apparatus according to claim 1, wherein said parent container (B1) includes a partition wall (d) extending from a position intervening between said first outlet (Q1) and said second outlet (Q2) to a position spaced a predetermined distance from the parent container side opposite to the side thereof provided with said first and second outlets, and said container pressing member (18) is provided with a spring member (21) capable of urging said pressing member (20) in the direction of said supporting member (19) while said supporting member (19) is kept in a fixed state.

3. A liquid separating apparatus according to claim 2, wherein said supporting member (19) is formed of a door member of a main body case (14).

4. A liquid separating apparatus according to claim 3, wherein said supporting member (19) is a transparent body.

5. A liquid separating apparatus according to claim 4, wherein said pressing member (20) is connected to said door member through an interlocking mechanism (22-26) so as to be moved in the direction opposite to the direction of pressurization as a result of the opening of the door member of said main body case (14).

6. A liquid separating apparatus according to anyone of claims 3 to 5, wherein said container pressing member (18) is adapted so as to press on said parent container (b1) under the biasing force of said spring member (21) by locking a handle (19a) attached to said door member.

7. A liquid separating apparatus according to claim 6, wherein said weight measuring unit (G) is adapted to detect the weight of said parent container by determining the total weight of a container pressing unit formed by utilizing said container pressing member (18) and subtracting a tare from the total weight consequently obtained.

## Patentansprüche

1. Vorrichtung zum Trennen von Flüssigkeiten, umfassend:
- ein Behälterhalteelement (11) zum Halten eines Weich-Stammbehälters (B1), welcher dazu angepaßt ist, darin Komponenten aufzubewahren, welche in mindestens drei Schichten zerlegt sind, das heißt, in eine obere Schicht (S1), eine Zwischenschicht (S3) und eine untere Schicht (S2), wobei der Weich-Stammbehälter (B1) an einer Seite davon vorgesehen ist, welche der oberen Schicht (S1) oder der unteren Schicht (S2) mit einer ersten Auslaßöffnung (Q1) und einer zweiten Auslaßöffnung (Q2) zugewandt ist,
- ein Behälterdruckelement (18), umfassend ein Stützelement (19) zum Stützen einer von zwei gegenüberliegenden Oberflächen des Stammbehälters (B1) und ein Druckelement (20) zum Drücken der anderen Oberfläche des Stammbehälters (B1);
- ein Grenzflächenerfassungselement (43) zum Erfassen einer Position des Flüssigkeitsniveaus der Zwischenschicht (S3) in dem Stammbehälter (B1) während einer Unter-Druck-Setzung des Stammbehälters durch das Behälterdruckelement (18), dadurch gekennzeichnet, daß diese ferner umfaßt:
- ein erstes Klemmelement (40a) und ein zweites Klemmelement (40b) zum unabhängigen Sperren der Flüssigkeitsströme, welche aus dem Stammbehälter und durch die erste und die zweite Auslaßöffnung (Q1, Q2) hindurch infolge einer Unter-Druck-Setzung des Stammbehälters (B1) durch das Druckelement (20) erzeugt werden; und
- eine Steuereinheit (C) zum Steuern der Auf-Zu-Zustände des ersten und zweiten Elements (40a, 40b) gemäß Signalen von dem Grenzflächenerfassungselement (43), wodurch die Position der Zwischenschicht (S3) bei einem im wesentlichen festen Niveau gehalten wird und die obere Schicht (S1) und die untere Schicht (S2) zu den vorbestimmten Auslaßöffnungen (Q1, Q2) geleitet werden, und eine Gewichtsmeßeinheit (G) zum Messen des Gewichts des Stammbehälters (B1); wobei die Steuereinheit (C) vor einer Unter-Druck-Setzung das durch eine Gewichtsmeßeinheit gemessene Gewicht des Stammbehälters (B1) mit einem ersten vorbestimmten Gewicht vergleicht, um eine Unter-Druck-Setzung zu ermöglichen, wenn das Gewicht des Stammbehälters (B1) gleich dem ersten vorbestimmten Gewicht ist, und während des Drückens des Behälters durch das Behälterdruckelement (18) das durch eine Gewichtsmeßeinheit (G) kontinuierlich erfaßte Gewicht des Stammbehälters (B1) mit einem zweiten vorbestimmten Gewicht vergleicht, um die Auf-Zu-Steuerung der Klemmelemente (40a, 40b) auf der Basis der Daten des Gewichts des Stammbehälters (B1) und der Position der Zwischenschicht (S3) zu bewirken, und die zwei Klemmelemente (40a, 40b) schließt, um die Flüssigkeitsströme anzuhalten, wenn eine vorbestimmte Zeit verstrichen ist, bzw., wenn das Gewicht des Stammbehälters (B1) gleich dem zweiten vorbestimmten Gewicht ist.

2. Vorrichtung zum Trennen von Flüssigkeiten nach Anspruch 1, wobei der Stammbehälter (B1) eine Trennwand (d) umfaßt, welche von einer Position zwischen der ersten Auslaßöffnung (Q1) und der zweiten Auslaßöffnung (Q2) zu einer Position verläuft, welche sich in einem vorbestimmten Abstand von der Stammbehälterseite gegenüber der Seite davon befindet, welche mit der ersten und zweiten Auslaßöffnung versehen ist, und das Behälterdruckelement (18) mit einem Federelement (21) versehen ist, welches in der Lage ist, das Druckelement (20) in die Richtung des Stützelements (19) zu drängen, während das Stützelement (19) in einem festen Zustand gehalten wird.

3. Vorrichtung zum Trennen von Flüssigkeiten nach Anspruch 2, wobei das Stützelement (19) aus einem Türelement eines Hauptkörpergehäuses (14) gebildet ist.

4. Vorrichtung zum Trennen von Flüssigkeiten nach Anspruch 3, wobei das Stützelement (19) ein transparenter Körper ist.

5. Vorrichtung zum Trennen von Flüssigkeiten nach Anspruch 4, wobei das Druckelement (20) über eine Verriegelungsvorrichtung (22 - 26) verbunden ist, um in der Gegenrichtung der Unter-Druck-Setzungs-Richtung als Ergebnis der Öffnung des Türelements des Hauptkörpergehäuses (14) bewegt zu werden.

6. Vorrichtung zum Trennen von Flüssigkeiten nach einem der Ansprüche 3 bis 5, wobei das Behälterdruckelement (18) dazu angepaßt ist, unter der Vorspannkraft des Federelements (21) durch Verriegeln eines an dem Türelement befestigten Griffs (19a) auf den Stammbehälter (B1) zu drücken.

7. Vorrichtung zum Trennen von Flüssigkeiten nach Anspruch 6, wobei die Gewichtsmeßeinheit dazu angepaßt ist, das Gewicht des Stammbehälters durch Bestimmen des Gesamtgewichts einer durch Verwenden des Behälterdruckelements (18) gebildeten Behälterdruckeinheit und Subtrahieren einer Tara von dem folglich erhaltenen Gesamtgewicht zu erfassen.

## Revendications

1. Appareil de séparation de liquides, comprenant :
- un élément (11) de retenue de récipient servant à retenir un récipient source souple (B1) adapté pour le stockage de composants fractionnés en au moins trois couches, c'est-à-dire une couche supérieure (S1), une couche intermédiaire (S3) et une couche inférieure (S2), ledit récipient source souple (B1) étant pourvu sur un de ses côtés faisant face soit à ladite couche supérieure (S1), soit à ladite couche inférieure (S2), d'un premier orifice de sortie (Q1) et d'un second orifice de sortie (Q2),
- un élément (18) de compression de récipient comprenant un élément de support (19) pour supporter l'une quelconque des deux surfaces opposées dudit récipient source (B1) et un élément de compression (20) servant à comprimer l'autre surface dudit récipient source (B1);
- un élément (43) de détection d'interface pour déterminer la position du niveau de liquide de ladite couche intermédiaire (S3) dans ledit récipient source (B1) au cours de la compression dudit récipient source par ledit élément (18) de compression de récipient, caractérisé en ce qu'il comprend de plus :
- un premier élément d'arrêt (40a) et un second élément d'arrêt (40b) pour bloquer indépendamment les écoulements de liquide provenant dudit récipient source et dans lesdits premier et second orifices de sortie (Q1, Q2) provoqués par la compression du récipient source (B1) par ledit élément de compression (20); et
- une unité de commande (C) pour commander les états ouvert-fermé desdits premier et second éléments (40a, 40b) en fonction de signaux provenant dudit élément (43) de détection d'interface, ce qui permet de conserver la position de ladite couche intermédiaire (S3) à un niveau sensiblement fixe et de guider ladite couche supérieure (S1) et ladite couche inférieure (S2) jusqu'aux orifices de sortie prédéterminés (Q1, Q2), et une unité (G) de pesage pour mesurer le poids dudit récipient source (B1); ladite unité de commande (C) comparant, avant compression, le poids dudit récipient source (B1) mesuré par l'unité (G) de pesage à un premier poids prédéterminé pour permettre la compression quand le poids dudit récipient source (B1) est égal au premier poids prédéterminé, et comparant, au cours du relâchement dudit récipient par ledit élément (18) de compression de récipient, le poids dudit récipient source (B1) mesuré en continu par l'unité (G) de pesage à un second poids prédéterminé pour exécuter la commande ouvert-fermé desdits éléments d'arrêt (40a, 40b) en se basant sur les données du poids dudit récipient source (B1) et de la position de ladite couche intermédiaire (S3), et fermant les deux éléments d'arrêt (40a, 40b) pour stopper les écoulements de liquides lorsqu'un temps prédéterminé s'est écoulé ou que le poids dudit récipient source (B1) est égal au second poids prédéterminé.

2. Appareil de séparation de liquides selon la revendication 1, dans lequel ledit récipient source (B1) inclut une paroi de séparation (d) s'étendant depuis une position se situant entre ledit premier orifice de sortie (Q1) et ledit second orifice de sortie (Q2) jusqu'à une position située à une distance prédéterminée du côté du récipient source opposé au côté de ce dernier pourvu desdits premier et second orifices de sortie, et ledit élément (18) de compression de récipient est pourvu d'un ressort (21) pouvant pousser ledit élément de compression (20) dans la direction dudit élément de support (19) tandis que ledit élément de support (19) est maintenu dans un état fixe.

3. Appareil de séparation de liquides selon la revendication 2, dans lequel ledit élément de support (19) est formé d'une porte d'un boîtier principal (14).

4. Appareil de séparation de liquides selon la revendication 3, dans lequel ledit élément de support (19) est un corps transparent.

5. Appareil de séparation de liquides selon la revendication 4, dans lequel ledit élément de compression (20) est relié à ladite porte grâce à un mécanisme de liaison (22-26) afin d'être déplacé dans le sens opposé au sens de compression conséquemment à l'ouverture de la porte dudit boîtier principal (14).

6. Appareil de séparation de liquides selon l'une quelconque des revendications 3 à 5, dans lequel ledit élément (18) de compression de récipient est adapté de façon à exercer une pression sur ledit récipient source (B1) sous la force de contrainte dudit ressort (21) en verrouillant une poignée (19a) fixée à ladite porte.

7. Appareil de séparation de liquides selon la revendication 6, dans lequel ladite unité (G) de pesage est adaptée pour détecter le poids dudit récipient source en déterminant le poids total d'une unité de compression de récipient formée en utilisant ledit élément (18) de compression de récipient et en soustrayant une tare au poids total ainsi obtenu.
